# EUROPEAN PATENT SPECIFICATION

(11) **EP 3 270 953 B1**
(45) Date of publication and mention of the grant of the patent: **07.07.2021**
(21) Application number: 16710978.4
(22) Date of filing: 18.03.2016
(51) Int. Cl.: A61K 39/00, A61K 38/21, A61K 45/06, A61K 31/7068

(54) **NOVEL TREATMENT METHOD**
NEUE BEHANDLUNGSMETHODEN
METHODES DES TRAITEMENT NOUVELLES

(30) Priority: 19.03.2015 GB 201504701
(43) Date of publication of application: 24.01.2018
(73) Proprietor: Idogen AB, 223 81 Lund (SE)
(72) Inventor: ERICSSON, Peter, SE 223 81 Lund (SE); HEDBYS, Lars, SE 223 81 Lund (SE); SALFORD, Leif, SE 223 81 Lund (SE); SJÖGREN, Hans-Olov, SE 223 81 Lund (SE)
(74) Representative: Kinch, Alison
(86) International application number: PCT/EP2016/056050
(87) International publication number: WO 2016/146842

(56) References cited:
- WO-A1-03/087347
- WO-A1-2008/147283
- WO-A1-2012/087234
- L. HSU ET AL: "Antidrug antibodies in psoriasis: a systematic review", BRITISH JOURNAL OF DERMATOLOGY, vol. 170, no. 2, 18 February 2014 (2014-02-18), pages 261-273, XP55271100, UK ISSN: 0007-0963, DOI: 10.1111/bjd.12654
- FALLARINO F ET AL: "Functional expression of idoleamine 2,3-dioxygenase by murine CD8alpha+ dendritic cells", 20020101, vol. 14, no. 1, 1 January 2002 (2002-01-01), pages 65-68, XP002954351,
- ALEXANDER A M ET AL: "Indoleamine 2,3-dioxygenase expression in transplanted NOD islets prolongs graft survival after adoptive transfer of biabetogenic splenocytes", 20020201, vol. 51, 1 February 2002 (2002-02-01), pages 356-365, XP002954352,
- HWU P ET AL: "Indoleamine 2,3-dioxygenase production by human dendritic cells results in the inhibition of T cell proliferation", 20000101, vol. 164, 1 January 2000 (2000-01-01), pages 3596-3599, XP002954350,
- WOO T ET AL: "Efficacy of Oral Collagen in Joint Pain - Osteoarthritis and Rheumatoid Arthritis", JOURNAL OF ARTHRITIS, vol. 06, no. 02, 1 January 2017 (2017-01-01), XP55506791, DOI: 10.4172/2167-7921.1000233
- Barnett: "AN - PREV199800170435 TI - Treatment of rheumatoid arthritis with oral type II collagen: Results of a multicenter, double-blind, placebo-controlled trial AU - Barnett Martha L; Kremer Joel M; St Clair E William; Clegg Daniel O; Furst Daniel; Weisman Michael; Fletcher Malcolm J F; Chason-Taber", , 3 February 1998 (1998-02-03), XP55506879, Retrieved from the Internet: URL:https://pdfs.semanticscholar.org/81d1/ c0bdb4fe215d5b371815dd5ee0fe9e1afc6f.pdf [retrieved on 2018-09-13]
- MIGUEL A. MAESTRO ET AL: "Vitamin D and Its Synthetic Analogs", JOURNAL OF MEDICINAL CHEMISTRY, vol. 62, no. 15, 27 March 2019 (2019-03-27), pages 6854-6875, XP55702171, ISSN: 0022-2623, DOI: 10.1021/acs.jmedchem.9b00208
- MASUDA SONOKO ET AL: "Promise of vitamin D analogues in the treatment of hyperproliferative conditions", MOLECULAR CANCER THERAPEUTICS, AMERICAN ASSOCIATION FOR CANCER RESEARCH, US, vol. 5, no. 4, 1 April 2006 (2006-04-01), pages 797-808, XP002491460, ISSN: 1535-7163, DOI: 10.1158/1535-7163.MCT-05-0539
- ZHONG-TIAN XUE ET AL: "An epigenetic mechanism for high, synergistic expression of indoleamine 2,3-dioxygenase 1 (IDO1) by combined treatment with zebularine and IFN-: Potential therapeutic use in autoimmune diseases", MOLECULAR IMMUNOLOGY, PERGAMON, GB, vol. 51, no. 2, 14 January 2012 (2012-01-14), pages 101-111, XP028421762, ISSN: 0161-5890, DOI: 10.1016/J.MOLIMM.2012.01.006 [retrieved on 2012-02-08]

### SEQUENCE LISTING

<110> Idogen AB
<120> Novel treatment method
<130> IDO-P1860PCT
<150> GB1504701.2
   <151> 2015-03-19
<160> 4
<170> Patent In version 3.5
<210> 1
   <211> 20
   <212> DNA
   <213> Homo sapiens
<400> 1
   ttgttctcat ttcgtgatgg 20
<210> 2
   <211> 20
   <212> DNA
   <213> Homo sapiens
<400> 2
   tactttgatt gcagaagcag 20
<210> 3
   <211> 22
   <212> DNA
   <213> Homo sapiens
<400> 3
   ctaattatgg acaggactga ac 22
<210> 4
   <211> 20
   <212> DNA
   <213> Homo sapiens
<400> 4
   agcaaagaat ttatagcccc 20

## Description

### Field of the invention

The invention relates to a method of treating mammalian subjects who develop anti-drug antibodies, especially antibodies against Factor VIII (FVIII). Specifically, it relates to the use of autologous antigen presenting cells (particularly dendritic cells) treated *ex-vivo* with an IDO inducing agent (such as zebularine) in the presence of a drug antigen (such as FVIII) for this purpose.

### Background of the invention

### Anti-drug antibodies

Haemophilia A (HA) is an X-chromosome linked bleeding disorder caused by a variety of mutations in the F8 gene encoding FVIII that interfere with the expression or pro-coagulant function of the translated protein. FVIII is expressed primarily in liver and endothelial vascular beds. Lacking sufficient pro-coagulant activity, haemophilia A patients are prone to bleeding episodes and their sequelae, including increased morbidity and mortality. The FVIII database currently identifies 2,015 unique FVIII variants based on 5,472 individual case reports. This vast array of point mutations (66.5%), deletions (23.2%) and others (duplication, polymorphism, insertions, indel and complex) leads to a variety of clinical outcomes. Patients can be treated acutely (on-demand) or prophylactically with either plasma-derived or recombinant FVIII.

A significant number of patients form neutralizing antibodies, termed "inhibitors", which block the activity of the administered FVIII because their immune systems have not been rendered fully tolerant to certain sequences of normal FVIII. Inhibitor development is currently the most significant treatment complication seen in patients with haemophilia.

At present, once inhibitors form, the only proven method for eradication is immune tolerance induction (ITI) through regular FVIII infusions but this treatment strategy fails in 10-20% of patients.

The problem of anti-drug antibodies is not limited to haemophiliacs with a defective FVIII. Anti-drug antibodies can develop in subjects treated with other drugs.

It is believed that the first step of an immune response against FVIII is recognition of FVIII by antigen presenting cells (APCs). Following endocytosis of FVIII by the APCs, FVIII is processed into small peptides which are then loaded on MHC class II molecules at the cell surface for presentation to FVIII-specific CD4⁺ T cells. The activation of these T cells requires additional signals provided by the APC. These signals are membrane-associated interactions between molecules such as CD40, CD80, CD83 and CD86 on the plasma membrane of the APC and CD28, CD154 and CTLA-4 on the T cell. In combination with these interactions, the APC signals to T cells via cytokines such as IL-12 or IL-10. The combination of signals determines the direction into which the activated T cell differentiates. T helper 1 (Th1) cells generally induce a cytotoxic immune response, Th2 a B-cell mediated antibody response and regulatory T cells are able to induce immunosuppression/tolerance by suppressing B- and T cell responses. Ultimately, these FVIII-specific T cells are able to activate FVIII-specific B-cells and induce affinity maturation and class-switching of immunoglobulin genes in B-cells. As a result, anti-FVIII antibody-secreting plasma cells and circulating FVIII-specific memory B-cells are generated, which are able to produce antibodies upon re-exposure to FVIII.

### Antigen presenting cells

Antigen presenting cells (APCs) are cells that display foreign antigens complexed with major histocompatibility complexes (MHCs) on their surfaces; they process antigens and present them to T cells. APCs fall into two categories: professional and non-professional, the professional APC being those that express MHC class II molecules. There are four main types of professional APCs: dendritic cells, macrophages, certain B-cells and certain activated epithelial cells. Interaction of APCs with T-cells occurs in the lymph nodes, to which APCs are directed through the effect of chemotaxis. Dendritic cells can be isolated and matured from a number of sources, such as bone marrow (particularly hematopoietic bone marrow progenitor cells) and blood (particularly peripheral blood mononuclear cells, of which some are immature and are expressing CD34). Depending on the circumstances, (for example, level of expression of costimulators such as CD80/86, CD40, or IDO1, PD-L1 or PD-L2 with suppressive activity), the interaction of dendritic cells and T cells in the lymph nodes can lead to an immune or a tolerant response. Thus, a key interaction involves DCs expressing IDO1 and producing immunosuppressive cytokines (e.g. IL-10 and TGF-β), which induces the T cells to adopt a regulatory phenotype. The regulatory T cells in their turn do not only suppress the effector T cells or naïve T cells in their microenvironment, but they also induce immature DCs to differentiate to tolerogenic rather than immunogenic DCs thereby creating a tolerogenic loop response.

### IDO

IDO1, indoleamine 2, 3-dioxygenase 1, is a haem-containing enzyme catalyzing the initial, rate-limiting step in tryptophan degradation that is known to have an important role in inducing immune tolerance both in experimental animals and in humans. The key role of IDO1 has been clearly identified, for example, during mammalian pregnancy. IDO1 levels are increased and in this way it is essential for induction of immune tolerance vs. the foetus, and experimental inhibition of IDO1 in mice has been shown to result in rejection of foetuses expressing different MHC molecules than their mother.

When intracellularly expressed by DCs or other antigen presenting cells, IDO1 functions as a natural immunoregulator by suppressing T cell responses which results in immune tolerance.

### Zebularine

Zebularine is a cytidine analogue containing a 2-(1H)-pyrimidinone ring which is an effective inhibitor of DNA methylation. Zebularine is very stable with a half-life of approximately 44 h at 37°C in PBS at pH 1.0 and ∼508 h at pH 7.0, making oral administration of the drug possible. Indeed, orally administered zebularine has been shown to cause demethylation and reactivation of a silenced and hypermethylated p16 gene in human bladder tumour cells grown in nude mice. Zebularine also appears to be minimally cytotoxic *in vitro* and *in vivo,* at used concentrations, although toxic at high concentrations. Zebularine is known to induce IDO and the mechanism by which IDO is upregulated by zebularine has been suggested to be through epigenetic upregulation of the IDO1 gene.

WO2008/147283 discloses the use of zebularine for the manufacturing of a medicament for the treatment of an auto-immune disorder or disease or immune rejection of transplants or gene therapeutically modified cells, wherein the treatment induces IDO.

WO2012/087234 discloses a composition comprising at least two compounds, each of which induce indolamine 2,3-dioxygenase (IDO), for the treatment of an autoimmune disorder or disease or suffering from immune rejection of organs, tissues, normal cells or gene therapeutically modified cells, wherein said IDO inducers have different mechanisms of action and give rise to a synergistic effect on the IDO level. It also discloses a method of inducing IDO in a cell culture comprising the steps of (a) providing isolated cells in a suitable medium, (b) adding such a composition, (c) incubating said isolated cells with the composition and (d) obtaining a cell culture in which IDO is induced. It also discloses a method of treating a mammal having an autoimmune disorder or disease or suffering from immune rejection of organs, tissues, normal cells or gene therapeutically modified cells, wherein the treatment induces IDO, comprising firstly a treatment *ex vivo* of cells derived from the treated mammal or from another mammal, with a therapeutically effective amount of such a composition in the presence of one or more antigens associated with a condition being treated, followed by the transfer of treated cells to the mammal being treated. Dhodapkar (2001) is a report of antigen-specific inhibition of effector T cell function in humans after injection of immature DCs.

Giannoukakis (2011) is a report of a Phase I (safety) study of autologous tolerogenic DCs in Type 1 diabetic patients.

Nittby (2013) is a report that zebularine induces long term survival of pancreatic islet allotransplants in Streptoxotocin treated diabetic rats.

None of the above prior art documents concern treatments for subjects who develop anti-drug antibodies.

The present invention addresses the need for a method of treating subjects who do not respond to the traditional method of ITI referred to above.

### Summary of the invention

The invention provides a method of inducing IDO (indoleamine 2,3-dioxygenase) in a cell culture comprising *ex-vivo* treating antigen presenting cells obtained from a mammal with an agent which induces IDO in said antigen presenting cells in the presence of a drug or an epitope containing fragment thereof, wherein the drug is Factor VIII or Factor IX and wherein the agent which induces IDO is selected from zebularine, 2'-deoxyzebularine, 5-fluoro-zebularine, 5-fluoro-2'-deoxyzebularine, 5-chloro-zebularine, 5-chloro-2'-deoxyzebularine, 5-bromo-zebularine, 5-bromo-2'-deoxyzebularine, 5-iodo-zebularine, 5-iodo-2'-deoxyzebularine, 5-methylpyrimidin-2-one, 5-Me-2'-deoxyzebularine, or mono, di or tri phosphates thereof or salts thereof, 5-methylcytidine, azacytidine or deoxyazacytidine, histone deacetylase inhibitors, vitamin D3 analogues, interferons, toll like receptor ligands, gonadotropin receptor signalling hormones, prostaglandin E2, soluble CTLA4 conjugates, TGF-beta, IL-10 and glucocorticoids.

The invention also provides antigen presenting cells in which IDO (indoleamine 2,3-dioxygenase) has been induced obtained by the claimed method, for use in a method of treating a mammal suffering from or susceptible to an immune reaction to drug treatment comprising the raising of anti-drug antibodies whereby according to said method said cells are transferred back to said mammal thereby to establish immune tolerance to the drug, wherein the drug is Factor VIII or Factor IX.

Without being limited by theory, according to the method of the present invention, subjects that have developed anti-drug antibodies are treated with autologous APCs that have been treated with zebularine (or other IDO inducer), which skews the phenotype of the APCs towards being tolerogenic. These tolerogenic APCs are primed *ex-vivo* with corresponding drug antigen and transferred back to the subject. The APCs migrate to the lymph nodes and spleen where they exert their tolerogenic effect upon other immune cells (including the generation of regulatory T cells and B-cells) leading to establishment of tolerance to the drug by initiation of a tolerogenic loop by regulatory T-cells skewing naïve DCs towards tolerogenic differentiation.

### Brief description of the figures

Figure 1 shows the suppressive effects of BMDCs cultured under various conditions on lymphocyte proliferation response to the antigen ovalbumin *in vitro* (see Example 1)
Figure 2 shows the suppressive effects of BMDCs cultured under various conditions on T cell proliferation response to the antigen ovalbumin *in vitro* (see Example 2)
Figure 3 shows the migration of BMDCs cultured with zebularine and PGE₂ after s.c. inoculation (see Example 3).
Figure 4 shows the proliferative response to restimulation *in vitro* of lymph node cells from immunized rats treated with BMDCs cultured under various conditions (see Example 4).
Figure 5 shows that treatment of BMDCs with zebularine increases their suppressive effects on the proliferative response to restimulation of FVIII-primed CD4+ T-cells to FVIII *in vitro* (see Example 5).
Figure 6 shows the effect of azacytidine on IDO1 gene expression (mRNA level) in THP-1 cells (see Example 8).
Figure 7 shows the effect of deoxyazacytidine on IDO1 gene expression (mRNA level) in THP-1 cells (see Example 9).

### Detailed description of the invention

### Definitions

The term "indolamine dioxygenase" or "IDO" as used herein means IDO1 (indoleamine 2,3-dioxygenase, EC 1.13.11.52) or IDO2 (indoleamine-pyrrole 2,3 dioxygenase-like 1, EC 1.13.11.-) these being two different proteins that can catabolize tryptophan, and can be expressed by APCs.

" Zebularine" means 2'-O-t-Butyldimethylsilyl-3'-O-[(di-isopropylamino)(2-cyanoethoxy)phosphino]-5'-O-(4,4'-dimethoxytrityl)-2(1H)-pyrimidinone-1-β-D-riboside, also known as 1-(p-D-Ribofuranosyl)-1,2-dihydropyrimidin-2-one or 2-Pyrimidone-1-β-D-riboside.

An analogue is a molecule that differs in chemical structure from a parent compound, for example a homolog (differing by an increment in the chemical structure, such as a difference in the length of an alkyl chain), a molecular fragment, a structure that differs by one or more functional groups, a change in ionization. Structural analogues are often found using quantitative structure activity relationships (QSAR), with techniques such as those disclosed in Remington (The Science and Practice of Pharmacology, 19th Edition (1995), chapter 28).

The term "synergistic effect" in the context of increasing IDO levels after the use of a combination of IDO inducers is intended to mean an increase in the IDO levels that is higher (preferably significantly higher) than the sum of the IDO levels achieved with each of the IDO inducers if used alone, said sum usually being referred to as an "additive effect".

### Mammals

Mammals that may be treated by methods according to the invention include humans, domestic animals and livestock animals, especially humans. Example domestic animals include cats and dogs. Example livestock animals include horses, cows, sheep and goats.

### APCs

APCs suitable for use in the method of the invention are APCs which express IDO, especially IDO1. Suitably the APCs are capable of inducing a tolerogenic loop, for example, by generating antigen specific regulatory T cells or B-cells.

Most suitably, the APCs are dendritic cells (DCs) such as bone marrow-derived dendritic cells (BMDCs), DCs generated from CD34+ hematopoetic progenitor cells (especially as present in peripheral blood) or DCs derived from peripheral blood mononuclear cells (PBMCs). DCs that are capable of proliferating are preferred. It appears possible to induce DCs derived from peripheral blood mononuclear cells (PBMCs) to proliferate by appropriate treatment (see e.g. Langstein, 1999).

In another aspect of the invention, the APCs are mesenchymal stem cells co-cultured with DCs.

### IDO inducers

Agents which induce IDO in methods according to the invention include zebularine or an analogue or salt thereof. Examples of zebularine analogues that may be used as IDO inducers according to the invention include 2'-deoxyzebularine, 5-fluoro-zebularine, 5-fluoro-2'-deoxyzebularine, 5-chloro-zebularine, 5-chloro-2'-deoxyzebularine, 5-bromo-zebularine, 5-bromo-2'-deoxyzebularine, 5-iodo-zebularine, 5-iodo-2'-deoxyzebularine, 5-methylpyrimidin-2-one, 5-Me-2'-deoxyzebularine, or mono, di or tri phosphates thereof. Examples of salts include salts that may be formed by zebularine with strong organic or inorganic acids such as HBr and HCl salts.

Further agents which induce IDO include other cytidine analogues (e.g. 5-methylcytidine, azacytidine or deoxyazacytidine), histone deacetylase inhibitors (e.g. valproic acid or salts thereof such as sodium valproate, trichostatin A or vorinostate (SAHA)), vitamin D3 analogues (e.g. calcitriol), interferon gamma analogues, other interferons (e.g. interferon alpha, interferon B1 or interferon-tau), toll like receptor ligands (e.g. CpG containing DNA oligonucleotides or lipopolysaccharides), gonadotropin receptor signalling hormones (e.g. recombinant human gonadotropin (rhCG) or prolactin), prostaglandin E2 analogues, IDO stabilizers (e.g. TGF-b or IL-10), soluble CTLA4 conjugates (e.g. CTLA4-Ig such as abatacept) and glycocorticoids (e.g. dexamethasone). Thus the agent which induces IDO may, for example, be selected from azacytidine and deoxyazacytidine.

In another aspect of the invention, two (or more) agents may be employed which both induce IDO. In one particular embodiment, one of the two IDO inducing agents is zebularine or an analogue or salt thereof. Suitably said two (or more inducers give rise to a synergistic effect and, for example, these two (or more) IDO inducing agents have different mechanisms of action. Said IDO inducers are selected from the group consisting of zebularine or an analogue or salt thereof, other cytidine analogues (e.g. 5-methylcytidine, azacytidine or deoxyazacytidine), histone deacetylase inhibitors (e.g. valproic acid or salts thereof such as sodium valproate, trichostatin A or vorinostate (SAHA)), vitamin D3 analogues (e.g. calcitriol), interferon gamma analogues, other interferons (e.g. interferon alpha, interferon B1 or interferon-tau), toll like receptor ligands (e.g. CpG containing DNA oligonucleotides or lipopolysaccharides), gonadotropin receptor signalling hormones (e.g. recombinant human gonadotropin (rhCG) or prolactin), prostaglandin E2 analogues, IDO stabilizers (e.g. TGF-b or IL-10), soluble CTLA4 conjugates (e.g. CTLA4-Ig such as abatacept) and glycocorticoids (e.g. dexamethasone). Specific combinations that may be mentioned include (i) zebularine and IFN-gamma, (ii) IFN-gamma and valproic acid, (iii) zebularine, IFN-gamma and valproic acid, (iv) human chorionic gonadotropin (hCG) and zebularine, (v) hCG and IFN-gamma, (vi) zebularine and IFN-A, (vii) zebularine, IFN-gamma and IFN-A, (viii) zebularine, IFN-gamma and TGF-beta, and (ix) zebularine, IFN-gamma, IFN-A, and TGF-beta. Two or more agents that induce IDO may be administered to the cells simultaneously, sequentially or separately (with an appropriate time separation) as will be appropriate for optimum effect.

### Obtaining APCs from the mammal

According to the invention, APCs such as DCs are first obtained from the mammal. One suitable method is to collect immature PBMCs by apheresis (optionally after mobilization of immature cells with standard doses of recombinant G-CSF). Another suitable method is to use Nycodenz centrifugation to separate low density monocytes after lyzing of the red blood cells. To obtain a population of proliferating DCs, immature CD34⁺ cells may be purified from PBMC using magnetic beads linked to anti-CD34 MAb (Dynal Biotech, Oslo, Norway). CD34⁺ cell purity can be confirmed by flow cytometry. Purified CD34⁺ cells are typically differentiated into DCs by by treatment with GM-CSF and IL-4. Thus, typically they are cultured at 1x10⁵ cells/ml in tissue culture flasks (Nunc, Roskilde, Denmark) in CellGro serum-free medium or RPMI medium containing 10% autologous plasma, 2 mM I- glutamine (JHR), 20-100 ng/ml GM-CSF 200-1000 U/m, IL-4 (R&D Systems, Minneapolis, MN, USA), optionally together with 5 - 20 ng/ml IL10 and 50 ng/ml Flt-3L (R&D Systems). Cells may also be cultured with foetal bovine serum or human serum AB. Cultures may be maintained at 37°C in humidified 5% CO₂ atmosphere for 14 days. As cellular expansion occurs, cell culture medium and cytokines are replenished (e.g. approximately every 4 days).

In some circumstances it may be possible to isolate BMDCs from a bone of the subject. DC cell differentiation can be induced and cells expanded as described above for CD34⁺ cells.

### Ex-vivo exposure to IDO inducer(s) and antigen

At day 12-14 immature DCs may be exposed to the IDO inducer or combination thereof (such as zebularine), together with an antigen preparation corresponding to the drug. The concentration of the IDO inducer (or combination) and the length of exposure can be selected for optimum IDO induction. A concentration of 1 µM - 1 mM e.g. 50-100 µM is exemplary.

The IDO inducer or combination thereof (such as zebularine) and the antigen preparation can be added to the DCs together or separately.

In order to achieve appropriate migration of the DCs to lymph nodes upon administering the cells into the patient, cells are typically treated for approximately 24 h with prostaglandin E2 (at concentration 1 - 10 µM) to induce required chemotactic receptors.

At day 15-17, DCs may be harvested, suspended in medium and stored frozen in liquid nitrogen until being released for use.

Typically, a quality control step will be performed prior to use. Quality control should include tests for viability, sterility and endotoxin and expression of IDO1. Additionally, expressions of PD-L1, PD-L2, the chemotactic receptor CCR7, level of expression of the costimulators CD80/CD86, CD40, and MHC-II are to be considered. Expression levels of these substances is an indicator that the DCs are tolerogenic.

### Administration of cells to the subject

Cells may be administered back to the mammal by various routes e.g. intravenously, subcutaneously or intracutaneously. The medium containing the cells is suitably containing human albumin as a cell-protecting protein. Typically an amount of 3-10 x 10⁶ cells/dose in 1-10 doses at weekly to bi-weekly intervals is administered. The treatment can be extended until the desired tolerance is achieved.

### Antigen

The antigen preparation corresponding to the drug may contain the drug in whole or part, said part comprising an epitope containing fragment thereof. Generally, a purified antigen will be used. Suitably, the antigen preparation corresponding to the drug resembles as closely as possible the drug which is being administered to the subject. In the case of FVIII, various recombinant drugs are available which are suitable for this purpose including the commercial products ReFacto AF, NovoSeven, Helixate NexGen, Kogenate Bayer, Advate, NovoEight, Nuwiq, Beriate, Beriate P, Feiba, Haemoctin, Hemofil, Monoclate - P, Octanate [LV], Optivate and Recombinate.

### Immune reactions and use of methods of the invention

The agent which induces IDO is for use in a method which is suitable for the treatment of mammalian subjects who develop an immune response comprising the raising of anti-drug antibodies to the blood factors FVIII or Factor IX

Such biological drugs may contain polysaccharide components.

The agent which induces IDO is for use in a method for the treatment of mammalian subjects who develop an immune reaction to Factor VIII or Factor IX, including the raising of anti-drug antibodies in a number of drug treatment situations, such as bleeding disorders (haemophilia A and B; respectively deficiency of FVIII and Factor IX)

### Advantages

The invention is, in at least some embodiments, expected to provide one or more of the following advantages:
- Successful raising of immune tolerance to drug, or reduction in immune intolerance to drug, e.g. as measured by reduced anti-drug antibody levels in the blood;
- A customised response to drug which is adapted for the individual subject. For example, each haemophilia A patient will have a unique response to native FVIII based on their own FVIII defect and other genetics such as HLA. Thus the autologous approach of the invention is capable of ensuring the subject's APCs become tolerogenic;
- Benign effect with low toxicity at required concentrations;
- Fairly rapid time to achievement of useful tolerance following initiation of the treatment regime.

### Examples

### Materials and methods

### Preparation of DCs for use in immunisation

Lewis rats are euthanized and femurs and tibia removed. The bones are then disinfected by immersing them in 70% ethanol for 1 min. Both ends of the bones are cut with sterile scissors and the bone marrow flushed and suspended in BMDC-medium (RPMI 1640 supplemented with 2% v/v normal Lewis rat serum, 10 mM HEPES, 100 U/ml penicillin, 100 µg/ml streptomycin and 50 µM 2-mercaptoethanol) using a 5 ml syringe with a 20-gauge needle. After one wash in BMDC-medium red blood cells are lysed and the bone marrow cells are then washed twice in BMDC-medium and passed through a 70-µm cell strainer.

Bone marrow cells (3.5 x 10⁶) are cultured at 37°C and 5% CO₂ in T25 cell culture flasks in 5 ml BMDC-medium supplemented with recombinant rat (rr)GM-CSF (5 ng/ml) and rrlL-4 (5 ng/ml). On day 3, 5 ml BMDC-medium supplemented with rrGM-CSF (5 ng/ml) and rrlL-4 (5 ng/ml) is added to each flask. On day 5, the medium and the non-adherent cells are aspirated and replaced with 5 ml fresh BMDC-medium containing rrGM-CSF (5 ng/ml), rrIL-4 (5 ng/ml) and zebularine (50 µM). Also, rrIL-10 (5 ng/ml) may be added here. On day 7, non-adherent cells and semi-adherent cells growing in clusters are harvested by gently tapping or flushing the flasks, centrifuged and dispersed in fresh BMDC-medium containing only rrGM-CSF (2.5 ng/ml) and zebularine (50 µM) and subcultured in fresh T25 culture flasks (2-3.5 x 10⁶/5 ml). In the case of separating nonadherent BMDCs from semiadherent BMDCs on day 7 (Example 5), nonadherent BMDCs are harvested 3-4 h after start of subculture and transferred to fresh T75 culture flasks. On day 8, prostaglandin E2 (PGE₂) (1-3 µM) may be added to the flasks (for upregulation of chemokine receptor 7 (CCR7) thereby enabling the BMDCs to migrate to lymph nodes). Also, the BMDCs can be pulsed with antigen by addition of the antigen to the flasks. On day 9 the cells are harvested by gently tapping or flushing the flasks. Cells that are going to be inoculated into rats are then extensively washed (2-3 times) in PBS. After washing, the cells (1-5x 10⁶) are resuspended in 0,2 ml PBS and inoculated s.c. in the thigh of the rat. BMDCs that are going to be tested in *in vitro* proliferation response assays are irradiated with 20 Gy before adding to the cultures.

In Examples 1-4, ovalbumin (OVA) was used as a model antigen.

In Example 5, Factor VIII (FVIII) was used as an antigen.

### In vitro proliferation response assay for evaluation of suppressive effects

Lewis rats were immunized one time at the tail base with 100 µg OVA emulsified in complete Freund's adjuvant (Examples 1-4), or twice with 150 lU/kg recombinant human FVIII with a 2-week-interval (Example 5). Inguinal lymph nodes were isolated from immunized rats seven days after the immunization. A standard proliferation response assay was performed in 96-well plates in a total volume of 150 µl/well (RPMI 1640 supplemented with 10% v/v heat inactivated fetal calf serum, 10 mM HEPES, 100 U/ml penicillin, 100 µg/ml streptomycin and 50 µM 2-mercaptoethanol) using a total lymph node cell population (Example 1 and 4) (100000/well) or using isolated CD4⁺ T cells (50000/well) with irradiated (20 Gy) syngeneic DCs (10000/well (Example 2) or 5000/well (Example 5)) as stimulators. To generate CD4⁺ T cells (≥98% purity), lymph node cells were stained with an antibody cocktail and the CD4⁺ T cells were isolated by negative selection using magnetic beads. To generate syngeneic Lewis DCs, naive spleen cells were isolated, and centrifuged over 14.5% Nycodenz after lyzing of the red blood cells. The low-density spleen cells were isolated from the gradient interface and from those the OX62⁺ DCs were isolated by positive selection using magnetic beads. Primed lymphocytes were stimulated by adding OVA (100 µg/ml) (Examples 1-4) or FVIII (1 µg/ml) (Example 5) to the cultures. Negative controls were set up with medium only (no OVA or FVIII).

After 3 days at 37°C in 5% CO₂, cultures were pulsed for the last 8 h with 0.5 µCi of [3H]thymidine. The cells were then harvested onto glass-fiber filters, and [3H]thymidine incorporation as a measure of T cell proliferation was measured using standard scintillation procedures. Experiments were performed in three or six replicates and the results were expressed in counts per minute (cpm) ± standard deviation (SD).

To assess the suppressive effects of the BMDCs on the proliferative response, irradiated (20 Gy), BMDCs (10000/well (Examples 1 and 2) or 5000/well (Example 5)) were added to the proliferation response assay.

BMDCs express the enzyme inducible nitric oxide synthase (iNOS) which has a strong suppressive effect on T cell proliferation. Expression of iNOS in BMDCs is induced by interferon gamma which is released from activated, proliferating T cells. We are not primarily interested in the suppressive effect of iNOS in our experiments. In fact, since the suppressive effect is so powerful we have to block the enzymatic iNOS activity by adding L-NIL (N⁶-(1-iminoethyl)-L-lysine dihydrochloride), a selective inhibitor of iNOS to ensure that the suppression is not only due to iNOS activity.

### Induction of IDO1 in THP-1 cells

THP-1 cells were harvested from appropriate number of T75 flasks. Cells were counted in a Bürker chamber, stained with 0.4% Trypan Blue. The appropriate number of cells for each experiment were transferred to a new tube and centrifuged at 1200 rpm, 5 min at room temperature (RT). The supernatant was discarded and fresh RPMI with 5% FBS were added to get a cell concentration of 2x10⁵ cells/mL. The compound was added to the five separate wells in 6-well plates such that five different final concentrations were obtained after adding the cells. A control was added to one well in the 6-well plate to which cells were added. 1x10⁶ cells per well were seeded out to achieve a total volume of 5 mL per well. The cells were incubated at 37°C in 5% CO₂ for 96 hours. After 96 hours the assay was stopped and the cells were lysed for the isolation of RNA.

### RNA extraction and quantification of gene expression by RT-qPCR

Total RNA was extracted from THP-1 cells using the RNA RNeasy Mini extraction kit (Qiagen), according to the manufacturer's instructions. A SuperScript® VILO™ RT-kit (Thermo Fisher) was used to reverse transcribe 1 µg of total RNA to cDNA and qPCR was performed. The primers used for amplification of the human IDO1 gene were 5'-TTGTTCTCATTTCGTGATGG-3' (forward; SEQ ID No. 1) and 5'-TACTTTGATTGCAGAAGCAG-3' (reverse; SEQ ID No. 2). The primers used for amplification of the endogenous reference gene for human HPRT1 were 5'-CTAATTATGGACAGGACTGAAC-3' (forward; SEQ ID No. 3) and 5'-AGCAAAGAATTTATAGCCCC-3' (reverse; SEQ ID No. 4). In brief, RT-qPCR was performed in 20 µl reaction, consisting 10 µl PowerUp SYBR Green Master Mix, 0.5 µM of each primer, 4 µl diluted cDNA for amplification of IDO1 and 1.5 µl diluted cDNA for amplification of HPRT1. An iCycler iQ real-time detection system (Stratagene, Mx3000P, La Jolla, CA, USA) with the following thermal profile: UDG incubation at 50 °C for 2 min, then denaturation at 95 °C for 5 min, followed by 45 cycles at 94 °C for 15s, 58 °C for 30 s, and 60 °C for 30 s, was used to perform thermocycling and real-time detection of PCR products. After amplification a melting curve analysis was performed. The RT-qPCR experiments were always run in triplicate. IDO1 was normalised to the geometric means of two HPRT1 reference gene, using the ΔCt method. Within-group comparisons were normalized to one control sample, using the ΔΔCt method.

### Example 1 - BMDCs cultured with zebularine (with or without PGE₂) suppress the proliferative response of lymph node cells in vitro

Rats were immunized at the tail base with 100 µg OVA emulsified in complete Freund's adjuvant. Seven days post immunization inguinal lymph nodes were isolated. Lymph node cells (LN cells, total lymph node population) were cultured in 96-well plates (100000 per well) and re-stimulated with OVA (100 µg/ml) or un-stimulated (control, no OVA) for 3 days with or without the addition of irradiated (20 Gy) BMDCs (10000 per well) cultured with zebularine (50 µM), with or without PGE₂. To ensure that the suppressive effect was not only due to iNOS activity, the iNOS inhibitor L-NIL (0.01 mg/ml) was added to some of the wells. After 3 days at 37°C in 5% CO₂, cultures were pulsed for the last 8 h with 0.5 µCi of [3H]thymidine.

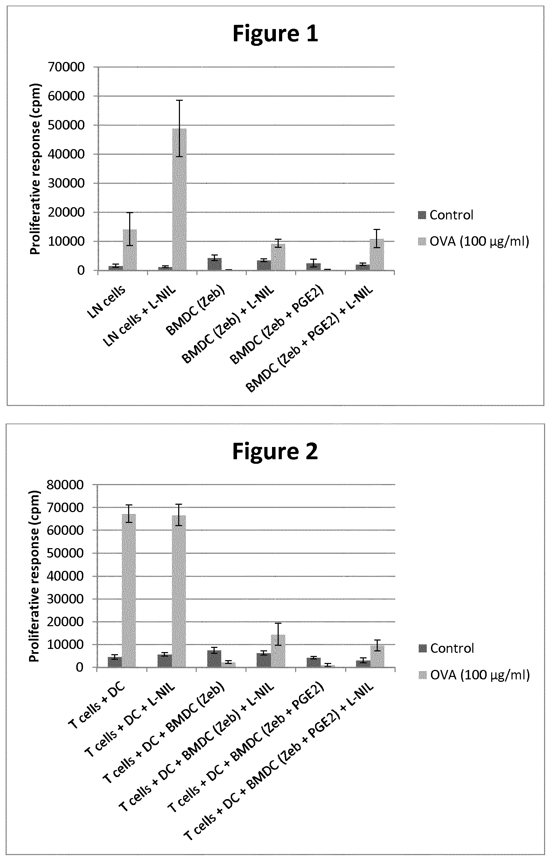

The results are expressed as the mean ± SD of sextuplicate wells (Figure 1).

The results demonstrate a complete suppression of the proliferative response of immune lymph node cells towards the defined antigen ovalbumin when added *in vitro* in the proportion 1:10. Culture of the DCs in the presence of PGE₂ does not reduce this effect. Based on the limited increase of lymph node cell response in the presence of the iNOS inhibitor L-NIL, only a minor part of the effect of the added DCs appears to be due to the expression of the enzyme iNOS that is under other circumstances known to be capable of a strong transient suppression of T lymphocyte reactivity *in vitro.* The results confirm that the DCs generated do exhibit the capacity to inhibit the response of lymph node cells to protein antigens *in vitro.*

### Example 2 - BMDCs cultured with zebularine (with or without PGE₂) suppress the proliferative response of T cells in vitro

Rats were immunized at the tail base with 100 µg OVA emulsified in complete Freund's adjuvant. Seven days post immunization CD4⁺ T cells were isolated from inguinal lymph nodes. Also, CD4⁺ T cells (50000 per well) were co-cultured with OX62⁺ DCs (isolated from spleens from untreated control Lewis rats) (10000 per well) in 96-well plates and restimulated with OVA (100 µg/ml) or un-stimulated (control, no OVA) for 3 days with or without the addition of BMDCs (10000 per well) cultured with zebularine (50 µM), with or without PGE₂. To ensure that the suppressive effect was not only due to iNOS activity, the iNOS inhibitor L-NIL (0.01 mg/ml) was added to some of the wells. After 3 days at 37°C in 5% CO₂, cultures were pulsed for the last 8 h with 0.5 µCi of [3H]thymidine.

The results are expressed as the mean ± SD of sextuplicate wells (Figure 2).

These results demonstrate that the admixed DCs are indeed capable of suppressing the response of pure CD4⁺ T lymphocytes (≥98%, with minimal contamination of B lymphocytes or non-lymphocyte cells). It is particularly important to inhibit those T cells, since they have a central role in executing the immune response of cytolytic effector cells, and helping the antibody producing B-cells to execute the production of antibodies. Note that in this *in vitro* experiment a type of activating spleen OX62⁺ DCs has to be added to present the antigen for pure T lymphocytes. Our suppressive DCs in this case suppress the activity of these activating DCs.

### Example 3 - BMDCs cultured with zebularine and PGE₂ migrate to draining lymph nodes after s.c. inoculation

Rats were immunized at the tail base with 100 µg OVA emulsified in complete Freund's adjuvant. Five days later rats were inoculated s.c. with 2x10⁶ CFSE-stained BMDCs, cultured with zebularine (50 µM) and PGE₂, in the thighs. Inguinal lymph nodes were isolated 72h post inoculation of BMDCs and low density lymph node cells were isolated by density centrifugation. Samples from the isolated low density cells were analyzed on a FACSCalibur for detection of CFSE-positive BMDCs.

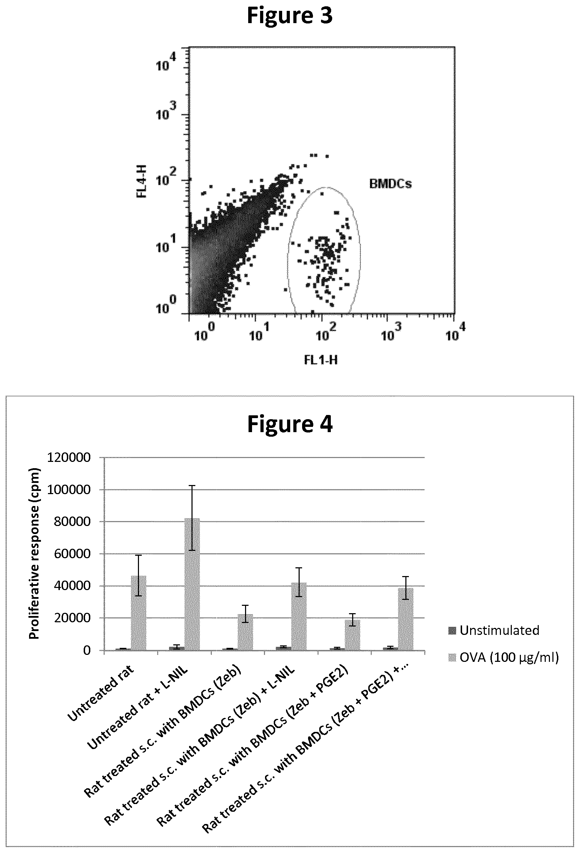

The results are shown in Figure 3.

An important feature for the in *vivo* effects of the tolerogenic DCs is that they exhibit the receptors required to be able to migrate to the lymph node draining the area of inoculation. It is the role of PGE₂ treatment of the DCs to enhance the expression of a main receptor (CCR7) of this type. These results show that the suppressive DCs do indeed migrate to the draining lymph node.

### Example 4 - Lymph node cells from immunized rats treated with BMDCs cultured with zebularine (with or without PGE₂) express reduced proliferative response to restimulation in vitro.

Rats were immunized at the tail base with 100 µg OVA emulsified in complete Freund's adjuvant. At the same time some of the rats received 2x10⁶ BMDCs cultured with zebularine (50 µM), with or without PGE₂ by s.c. inoculation in the thighs. Seven days post immunization inguinal lymph nodes were isolated. Lymph node cells (LN cells, total lymph node population) were cultured in 96-well plates (100000 per well) and restimulated with OVA (100 µg/ml) for 3 days. To ensure that the suppressive effect was not only due to iNOS activity, the iNOS inhibitor L-NIL (0.01 mg/ml) was added to some of the wells. After 3 days at 37°C in 5% CO₂, cultures were pulsed for the last 8 h with 0.5 µCi of [3H]thymidine.

The results are expressed as the mean ± SD of sextuplicate wells (Figure 4).

These *in vivo* results demonstrate that the DCs inhibit the responsiveness of the lymph node lymphocytes when they are inoculated simultaneously with immunization but at a different site although drained to the same lymph node. The inhibitory effect was not due to iNOS activity.

### Example 5 - Treatment of BMDCs with zebularine increases their suppressive effects on the proliferative response to restimulation of FVIII-primed CD4+ T-cells to FVIII in vitro

Rats were immunized twice s.c. at the tail base with 150 IU/kg human FVIII (Advate) mixed with 1 µg LPS with a 2-week-interval. Seven days post immunization CD4⁺ T cells were isolated from inguinal draining lymph nodes. CD4⁺ T cells (50000 per well) were co-cultured with OX62⁺ DCs (isolated from spleens from untreated control Lewis rats) (5000 per well) in 96-well plates and re-stimulated with FVIII (Advate, 1 µg/ml) or un-stimulated for 3 days with or without the addition of semiadherent ("Semiadh") or nonadherent ("Nonadh") BMDCs in ratio 1:100 (BMDCs: T cells, 500 BMDCs per well) or 1:10 (BMDCs: T cells, 5000 BMDCs per well) cultured with or without zebularine ("Zeb") (50 µM). To ensure that the recorded suppressive effect was not only due to iNOS activity, the iNOS inhibitor L-NIL (0.01 mg/ml) was added to some of the wells. After 3 days at 37°C in 5% CO₂, cultures were pulsed for the last 8 h with 0.5 µCi of [3H]thymidine.

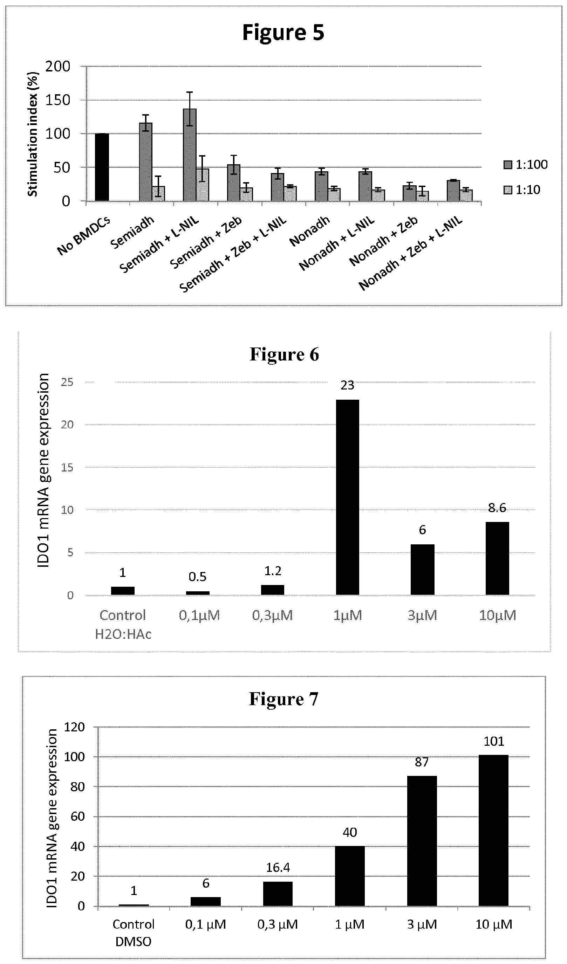

The results are expressed as percent stimulation index. The stimulation index (the FVIII-stimulated proliferative response divided by the non-stimulated proliferative response) was calculated for each sample. The stimulation index for wells without added BMDCs was considered as 100%. The results are expressed as the mean ± SD of triplicate wells (Figure 5).

These results demonstrate that the admixed DCs are indeed capable of suppressing the proliferative response of FVIII-primed CD4⁺ T lymphocytes (with minimal contamination of B lymphocytes or non-lymphocyte cells) to restimulation with FVIII *in vitro.* Also, these results demonstrate that treatment of DCs with zebularine increases these suppressive effects.

### Example 6 - Suppression of the immune response induced in rats immunized with human FVIII by i.v. transfer of dendritic cells expressing IDO1 induced in vitro

After harvest of bone marrow cells from adult rats, differentiation to immature dendritic cells is induced by culturing the cells in GM-CSF and IL-4. IDO1-expressing tolerogenic rat dendritic cells are generated *in vitro* by treatment of the immature dendritic cells with zebularine alone or with combinations of IFN-gamma and zebularine or other IDO-inducers (IFN-alpha, valproic acid, TGF-beta).

Adult rats are immunized twice intravenously with 150 IU/kg human FVIII mixed with 1 µg LPS with a 2-week-interval, known to induce both a detectable T-cell and a B-cell immune response to human FVIII. In one group of rats, three doses of *in vitro*-generated tolerogenic syngeneic DCs loaded with human FVIII are administered i.v., starting one week after last immunization. A control group of rats is similarly immunized but do not receive any i.v. transfer of dendritic cells. Animals are monitored for antibodies to human FVIII by ELISA and for anti-human FVIII T-cell responsiveness by assays for T-cell proliferation (thymidine incorporation assay) and cytokine production by ELISA (IL-2, IL-4, IL-5, IL-17, IFN-gamma, TNF-alpha, and IL-6).

Three doses of IDO1-expressing dendritic cells are expected to suppress T-cell response to human FVIII as assayed *in vitro* and also to suppress the antibody response to human FVIII. The suppressive effect of the combinations of IFN-gamma and zebularine or another IDO1-inducer is expected to be stronger due to a synergistic effect on the IDO1 induction by the combination of IFN-gamma and the respective inducer. Also the induction of a high affinity transporter of tryptophan in the dendritic cells by IFN-gamma (Bhutia, 2015) should cause a stronger suppressive effect on the immune T-cells that are unable to express this high affinity transporter.

### Example 7 - Demonstration in vitro of a suppressive effect of IDO1-expressing autologous dendritic cells on the reactivity of T-cells of an adult patient with Haemophilia A who has developed FVIII antibodies (inhibitors)

CD34⁺ PBMCs are isolated from an adult Haemophilia A patient who has developed antibodies (inhibitors) to FVIII. The cells are expanded *in vitro* by culture with SCF, Flt3L and differentiated to immature DC by GM-CSF and IL-4. The immature DCs are loaded with FVIII and exposed for 3 days to zebularine (50 µM) alone, or a combination of IFN-gamma and zebularine or IFN-alpha, or valproic acid, or TGF-beta, respectively, to induce expression of IDO1 and tolerogenic function.

On the day of testing of the generated tolerogenic DCs, autologous CD4⁺T-cells and monocytes are isolated separately from peripheral blood and purified (≥95%) by magnetic bead separation. T-cell responsiveness of the patient to FVIII *in vitro* is assayed by exposing 50,000 CD4⁺ T-cells to FVIII at concentration of 0, 0.01, 0.1, 0.3 and 1 µg/ml in the presence of 5,000 monocytes to present the FVIII. The T-cell proliferative response is measured by ³H-thymidine incorporation during the final 8 h of a 3-6 day incubation period. Release of cytokines (IL2, IL4, IL5, IL6, IL17, IFN-gamma, TNF-alpha) to culture medium is assayed by ELISA. Induction of transformation of the T-cells into Tregs by influence of the IDO1-expressing DCs is assayed in FACS by determining frequencies and numbers of FOX-P3⁺ CD4⁺cells.

The generated tolerogenic DCs are expected to inhibit the CD4⁺ T-cell proliferative response and their production/release of cytokines. Demonstration of a strong inhibition of the Th2 type cytokines will indicate a probable mechanism of a suppressive effect of a B-cell antibody response via suppression of Th2 and will predict that the DCs should have a suppressive effect on the anti-FVIII antibody production *in vivo.*

### Example 8 - Induction of IDO1 in THP-1 cells with azacytidine

The procedure under *"Induction of IDO1 in THP-1 cells"* was followed such that the final concentrations set out in Table 1 were obtained.

**Table 1: Final concentrations of crystalline 5-azacytidine in 5 mL wells**

| Sample No. | Compound | Final Concentration |
|---|---|---|
| 1 | HAc/H₂O 1:1 v/v (control) | 0.5 % v/v |
| 2 | 5-azacytidine | 0.1 micromolar |
| 3 | 5-azacytidine | 0.3 micromolar |
| 4 | 5-azacytidine | 1 micromolar |
| 5 | 5-azacytidine | 3 micromolar |
| 6 | 5-azacytidine | 10 micromolar |

Following RNA extraction and quantification of gene expression by RT-qPRC, as described above, induction of IDO1 gene expression using Samples 1 to 6 was investigated.

**Table 2: Results of 5-azacytidine samples on IDO1 expression**

| Sample No. | Concentration (micromolar) | IDO1 expression (fold change) |
|---|---|---|
| 1 | control | 1 |
| 2 | 0.1 | 0.5 |
| 3 | 0.3 | 1.2 |
| 4 | 1 | 23 |
| 5 | 3 | 6 |
| 6 | 10 | 8.6 |

The results are also shown in Figure 6. The results indicate that 5-azacytidine alone increases IDO1 gene expression by up to 23 fold. The highest level of IDO induction was observed at 1 micromolar concentration of 5-azacytidine.

### Example 9 - Induction of IDO1 in THP-1 cells with deoxyazacytidine

The procedure under *"Induction of IDO1 in THP-1 cells"* was followed such that the final concentrations set out in Table 3 were obtained.

**Table 3: Final concentrations of 5-aza-2'-deoxycytidine (decitabine) in 5 mL wells**

| Sample No. | Compound | Final Concentration |
|---|---|---|
| 7 | DMSO (control) | 0.5 % v/v |
| 8 | decitabine | 0.1 micromolar |
| 9 | decitabine | 0.3 micromolar |
| 10 | decitabine | 1 micromolar |
| 11 | decitabine | 3 micromolar |
| 12 | decitabine | 10 micromolar |

Following RNA extraction and quantification of gene expression by RT-qPRC, as described above, induction of IDO1 gene expression using Samples 7 to 12 was investigated.

**Table 4: Results of decitabine samples on IDO1 expression**

| Sample No. | Concentration (micromolar) | IDO1 expression (fold change) |
|---|---|---|
| 7 | control | 1 |
| 8 | 0.1 | 6 |
| 9 | 0.3 | 16.4 |
| 10 | 1 | 40 |
| 11 | 3 | 87 |
| 12 | 10 | 101 |

The results are also shown in Figure 7. The results indicate that 5-aza-2'-deoxycytidine alone increases IDO1 gene expression by up to 101 fold. The highest level of IDO induction was observed at 10 micromolar concentration of decitabine.

### References

Dhodapkar, M. et al., J. Exp. Med., 2001, 193(2), 233-238
Giannoukakis, N. et al., Diabetes Care, 2011, 34, 2026-2032
Nittby, H. et al., PLOS ONE, 2013, 8(8), 1-8
Langstein, J. Blood, 1999, 94, 3161-3168
Bhutia, Y. et al., Biochim Biophys Acta, 2015, 1848, 453-462

### Abbreviations

- HA: Haemophilia A
- ITI: Immune tolerance induction
- DC: Dendritic cell
- APC: Antigen presenting cell
- IDO: Indolamine dioxygenase
- L-NIL: N⁶-(1-iminoethyl)-L-lysine dihydrochloride
- MHC: Major histocompatibility complex
- IFN: Interferon
- DNA: Deoxyribonucleic acid
- PBS: Phosphate-buffered saline
- BMDC: Bone marrow-derived dendritic cell
- PBMC: Peripheral blood mononuclear cell
- OVA: Ovalbumin
- CFSE: Carboxyfluorescein succinimidyl ester
- FVIII: Factor VIII
- iNOS: inducible nitric oxide synthase
- s.c.: subcutaneous
- hCG: human chorionic gonadotropin
- EGF: epidermal growth factor
- HGF: hepatocyte growth factor
- KGF: keratinocyte growth factor
- TGF: tissue growth factor
- TGF-b: TGF-beta
- TNF: tumour necrosis factor
- G-CSF: granulocyte colony stimulating factor
- GM-CSF: granulocyte macrophage colony stimulating factor
- EPO: erythropoietin
- Ig: immunoglobulin
- IFN-A: interferon alpha
- MAb: monoclonal antibody
- rr: rat recombinant
- FACS: fluorescence activated cell sorting
- TNF-alpha: tumour necrosis factor-alpha
- Flt3L: FMS-like tyrosine kinase 3 ligand
- SCF: stem cell factor
- µ: micro
- ELISA: enzyme-linked immunosorbent assay
- Tregs: regulatory T-cells
- IL: Interleukin
- i.v.: intravenous
- FBS: foetal bovine serum
- RNA: ribonucleic acid
- HAc: acetic acid
- LN: lymph node
- SD: standard deviation

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

## Claims

1. A method of inducing IDO (indoleamine 2,3-dioxygenase) in a cell culture comprising *ex-vivo* treating antigen presenting cells obtained from a mammal with an agent which induces IDO in said antigen presenting cells in the presence of a drug or an epitope containing fragment thereof, wherein the drug is Factor VIII or Factor IX and wherein the agent which induces IDO is selected from zebularine, 2'-deoxyzebularine, 5-fluoro-zebularine, 5-fluoro-2'-deoxyzebularine, 5-chloro-zebularine, 5-chloro-2'-deoxyzebularine, 5-bromo-zebularine, 5-bromo-2'-deoxyzebularine, 5-iodo-zebularine, 5-iodo-2'-deoxyzebularine, 5-methylpyrimidin-2-one, 5-Me-2'-deoxyzebularine, or mono, di or tri phosphates thereof or salts thereof, 5-methylcytidine, azacytidine or deoxyazacytidine, histone deacetylase inhibitors, vitamin D3 analogues, interferons (IFN), toll like receptor ligands, gonadotropin receptor signalling hormones, prostaglandin E2, soluble CTLA4 conjugates, TGF-beta, IL-10 and glucocorticoids.

2. Antigen presenting cells in which IDO (indoleamine 2,3-dioxygenase) has been induced obtained by the method of claim 1, for use in a method of treating a mammal suffering from or susceptible to an immune reaction to drug treatment comprising the raising of anti-drug antibodies whereby according to said method said cells are transferred back to said mammal thereby to establish immune tolerance to the drug, wherein the drug is Factor VIII or Factor IX.

3. A method according to claim 1 or antigen presenting cells for use according to claim 2, wherein the antigen presenting cells are dendritic cells.

4. A method or antigen presenting cells for use according to claim 3 wherein the dendritic cells are bone marrow-derived dendritic cells, dendritic cells generated from CD34+ hematopoietic progenitor cells or dendritic cells generated from peripheral blood mononuclear cells.

5. A method or antigen presenting cells for use according to any one of claims 1 to 4, wherein the mammal is a human.

6. A method or antigen presenting cells for use according to any one of claims 1 to 5, wherein the agent which induces IDO is zebularine, 2'-deoxyzebularine, 5-fluoro-zebularine, 5-fluoro-2'-deoxyzebularine, 5-chloro-zebularine, 5-chloro-2'-deoxyzebularine, 5-bromozebularine, 5-bromo-2'-deoxyzebularine, 5-iodo-zebularine, 5-iodo-2'-deoxyzebularine, 5-methylpyrimidin-2-one, 5-Me-2'-deoxyzebularine, or mono, di or tri phosphates thereof or salts thereof.

7. A method or antigen presenting cells for use according to claim 6 wherein the agent which induces IDO is zebularine.

8. A method or antigen presenting cells for use according to any one of claims 1 to 5, wherein the agent which induces IDO is selected from azacytidine and deoxyazacytidine.

9. A method or antigen presenting cells for use according to any one of claims 1 to 5, wherein the agent which induces IDO is TGF-beta.

10. A method or antigen presenting cells for use according to any one of claims 1 to 5, wherein the agent which induces IDO is a vitamin D3 analogue.

11. A method or antigen presenting cells for use according to any one of claims 1 to 10, wherein two or more agents which induce IDO are used to induce IDO.

12. A method or antigen presenting cells for use according to claim 11, wherein the two or more agents are selected from (i) zebularine and IFN-gamma, (ii) IFN-gamma and valproic acid, (iii) zebularine, IFN-gamma and valproic acid, (iv) human chorionic gonadotropin (hCG) and zebularine, (v) hCG and IFN-gamma, (vi) zebularine and IFN-A, (vii) zebularine, IFN-gamma and IFN-A, (viii) zebularine, IFN-gamma and TGF-beta, and (ix) zebularine, IFN-gamma, IFN-A, and TGF-beta.

## Patentansprüche

1. Verfahren zum Induzieren von IDO (Indolamin-2,3-dioxygenase) in einer Zellkultur, umfassend ex-vivo-Behandlung von von einem Säugetier erhaltenen antigenpräsentierenden Zellen mit einem Mittel, das IDO in den antigenpräsentierenden Zellen induziert, in der Gegenwart eines Arzneimittels oder eines ein Epitop enthaltenden Fragments davon, wobei das Arzneimittel Faktor VIII oder Faktor IX ist und wobei das Mittel, das IDO induziert, ausgewählt ist aus Zebularin, 2'-Desoxyzebularin, 5-Fluorzebularin, 5-Fluor-2'-desoxyzebularin, 5-Chlorzebularin, 5-Chlor-2'-desoxyzebularin, 5-Bromzebularin, 5-Brom-2'-desoxyzebularin, 5-Iodzebularin, 5-Iod-2'-desoxyzebularin, 5-Methylpyrimidin-2-on, 5-Me-2'-desoxyzebularin oder Mono-, Di- oder Triphosphaten davon oder Salzen davon, 5-Methylcytidin, Azacytidin oder Desoxyazacytidin, Histondeacetylaseinhibitoren, Vitamin-D3-Analoga, Interferonen (IFN), Toll-like-Rezeptor-Liganden, Gonadotropin-Rezeptor-Signalhormonen, Prostaglandin E2, löslichen CTLA4-Konjugaten, TGF-beta, IL-10 und Glucocorticoiden.

2. Antigenpräsentierende Zellen, in denen IDO (Indolamin-2,3-Dioxygenase) induziert worden ist, erhalten nach dem Verfahren aus Anspruch 1, zur Verwendung in einem Verfahren zum Behandeln eines Säugetiers, das an einer Immunreaktion gegen Arzneimittelbehandlung leidet oder dafür anfällig ist, umfassend das Züchten von Anti-Arzneimittel-Antikörpern, wobei gemäß dem Verfahren die Zellen zurück in das Säugetier übertragen werden, wodurch eine Immuntoleranz gegenüber dem Arzneimittel aufgebaut wird, wobei das Arzneimittel Faktor VIII oder Faktor IX ist.

3. Verfahren nach Anspruch 1 oder antigenpräsentierende Zellen zur Verwendung nach Anspruch 2, wobei die antigenpräsentierenden Zellen dendritische Zellen sind.

4. Verfahren oder antigenpräsentierende Zellen zur Verwendung nach Anspruch 3, wobei die dendritischen Zellen aus Knochenmark gewonnene dendritische Zellen, aus CD34⁺ hämatopoetischen Vorläuferzellen erzeugte dendritische Zellen oder aus peripheren mononukleären Blutzellen erzeugte dendritische Zellen sind.

5. Verfahren oder antigenpräsentierende Zellen zur Verwendung nach einem der Ansprüche 1 bis 4, wobei das Säugetier ein Mensch ist.

6. Verfahren oder antigenpräsentierende Zellen zur Verwendung nach einem der Ansprüche 1 bis 5, wobei das Mittel, das IDO induziert, Zebularin, 2'-Desoxyzebularin, 5-Fluorzebularin, 5-Fluor-2'-desoxyzebularin, 5-Chlorzebularin, 5-Chlor-2'-desoxyzebularin, 5-Bromzebularin, 5-Brom-2'-desoxyzebularin, 5-Iodzebularin, 5-Iod-2'-desoxyzebularin, 5-Methylpyrimidin-2-on, 5-Me-2'-desoxyzebularin oder Mono-, Di- oder Triphosphate davon oder Salze davon ist.

7. Verfahren oder antigenpräsentierende Zellen zur Verwendung nach Anspruch 6, wobei das Mittel, das IDO induziert, Zebularin ist.

8. Verfahren oder antigenpräsentierende Zellen zur Verwendung nach einem der Ansprüche 1 bis 5, wobei das Mittel, das IDO induziert aus Azacytidin und Desoxyazacytidin ausgewählt ist.

9. Verfahren oder antigenpräsentierende Zellen zur Verwendung nach einem der Ansprüche 1 bis 5, wobei das Mittel, das IDO induziert, TGF-beta ist.

10. Verfahren oder antigenpräsentierende Zellen zur Verwendung nach einem der Ansprüche 1 bis 5, wobei das Mittel, das IDO induziert, ein Vitamin-D3-Analogon ist.

11. Verfahren oder antigenpräsentierende Zellen zur Verwendung nach einem der Ansprüche 1 bis 10, wobei zwei oder mehr Mittel, die IDO induzieren, eingesetzt werden, um IDO zu induzieren.

12. Verfahren oder antigenpräsentierende Zellen zur Verwendung nach Anspruch 11, wobei die zwei oder mehr Mittel ausgewählt sind aus (i) Zebularin und IFN-gamma, (ii) IFN-gamma und Valproinsäure, (iii) Zebularin, IFN-Gamma und Valproinsäure, (iv) menschliches Choriongonadotropin (hCG) und Zebularin, (v) hCG und IFN-Gamma, (vi) Zebularin und IFN-A, (vii) Zebularin, IFN-Gamma und IFN-A, (viii) Zebularin, IFN-Gamma und TGF-Beta und (ix) Zebularin, IFN-Gamma, IFN-A und TGF-Beta.

## Revendications

1. Procédé d'induction d'IDO (indoleamine 2,3-dioxygénase) dans une culture de cellules comprenant le traitement ex *vivo* de cellules présentatrices d'antigène obtenues à partir d'un mammifère avec un agent qui induit IDO dans lesdites cellules présentatrices d'antigène en présence d'un médicament ou d'un épitope contenant un fragment de celui-ci, dans lequel le médicament est le facteur VIII ou le facteur IX et dans lequel l'agent qui induit IDO est choisi parmi la zébularine, la 2'-désoxyzébularine, la 5-fluoro-zébularine, la 5-fluoro-2'-désoxyzébularine, la 5-chloro-zébularine, la 5-chloro-2'-désoxyzébularine, la 5-bromo-zébularine, la 5-bromo-2'-désoxyzébularine, la 5-iodo-zébularine, la 5-iodo-2'-désoxyzébularine, la 5-méthylpyrimidin-2-one, la 5-Me-2'-désoxyzébularine, ou des mono, di ou triphosphates de celles-ci ou des sels de celles-ci, la 5-méthylcytidine, l'azacytidine ou la désoxyazacytidine, des inhibiteurs d'histone désacétylase, des analogues de vitamine D3, des interférons (IFN), des ligands de récepteur de type Toll, des hormones de signalisation des récepteurs des gonadotrophines, la prostaglandine E2, des conjugués de CTLA4 solubles, TGF-bêta, IL-10 et des glucocorticoïdes.

2. Cellules présentatrices d'antigène dans lesquelles IDO (indoleamine 2,3-dioxygénase) a été induite, obtenues par le procédé selon la revendication 1, pour utilisation dans un procédé de traitement d'un mammifère souffrant ou susceptible d'une réaction immunitaire à un traitement médicamenteux comprenant l'induction d'anticorps anti-médicament où, selon ledit procédé, lesdites cellules sont transférées de retour vers ledit mammifère de façon à établir une tolérance immunitaire au médicament, où le médicament est le facteur VIII ou le facteur IX.

3. Procédé selon la revendication 1 ou cellules présentatrices d'antigène pour utilisation selon la revendication 2, où les cellules présentatrices d'antigène sont des cellules dendritiques.

4. Procédé ou cellules présentatrices d'antigène pour utilisation selon la revendication 3, où les cellules dendritiques sont des cellules dendritiques dérivées de moelle osseuse, des cellules dendritiques générées à partir de cellules progénitrices hématopoïétiques CD34+ ou des cellules dendritiques générées à partir de cellules mononucléaires de sang périphérique.

5. Procédé ou cellules présentatrices d'antigène pour utilisation selon l'une quelconque des revendications 1 à 4, où le mammifère est un humain.

6. Procédé ou cellules présentatrices d'antigène pour utilisation selon l'une quelconque des revendications 1 à 5, où l'agent qui induit IDO est la zébularine, la 2'-désoxyzébularine, la 5-fluoro-zébularine, la 5-fluoro-2'-désoxyzébularine, la 5-chloro-zébularine, la 5-chloro-2'-désoxyzébularine, la 5-bromo-zébularine, la 5-bromo-2'-désoxyzébularine, la 5-iodo-zébularine, la 5-iodo-2'-désoxyzébularine, la 5-méthylpyrimidin-2-one, la 5-Me-2'-désoxyzébularine, ou des mono, di ou triphosphates de celles-ci ou des sels de celles-ci.

7. Procédé ou cellules présentatrices d'antigène pour utilisation selon la revendication 6 où l'agent qui induit IDO est la zébularine.

8. Procédé ou cellules présentatrices d'antigène pour utilisation selon l'une quelconque des revendications 1 à 5, où l'agent qui induit IDO est choisi parmi l'azacytidine et la désoxyazacytidine.

9. Procédé ou cellules présentatrices d'antigène pour utilisation selon l'une quelconque des revendications 1 à 5, où l'agent qui induit IDO est TGF-bêta.

10. Procédé ou cellules présentatrices d'antigène pour utilisation selon l'une quelconque des revendications 1 à 5, où l'agent qui induit IDO est un analogue de vitamine D3.

11. Procédé ou cellules présentatrices d'antigène pour utilisation selon l'une quelconque des revendications 1 à 10, où deux ou plus de deux agents qui induisent IDO sont utilisés pour induire IDO.

12. Procédé ou cellules présentatrices d'antigène pour utilisation selon la revendication 11, où les deux ou plus de deux agents sont choisis parmi (i) la zébularine et IFN-gamma, (ii) IFN-gamma et l'acide valproïque, (iii) la zébularine, IFN-gamma et l'acide valproïque, (iv) la gonadotrophine chorionique humaine (hCG) et la zébularine, (v) hCG et IFN-gamma, (vi) la zébularine et IFN-A, (vii) la zébularine, IFN-gamma et IFN-A, (viii) la zébularine, IFN-gamma et TGF-bêta, et (ix) la zébularine, IFN-gamma, IFN-A et TGF-bêta.
